# EUROPÄISCHE PATENTANMELDUNG

(11) **EP 0 545 206 A1**
(43) Veröffentlichungstag der Anmeldung: **09.06.1993**
(21) Anmeldenummer: 92119923.8
(22) Anmeldetag: 23.11.1992
(51) Int. Cl.: C07D 239/54, C07D 239/96, A01N 43/54

(54) **N-Aryl-Stickstoffheterocyclen und ihre Verwendung als Herbizide**

(30) Priorität: 04.12.1991 DE 4139952
(71) Anmelder: BAYER AG, 51368 Leverkusen (DE)
(72) Erfinder: Schallner, Otto, Dr., W-4019 Monheim (DE); Santel, Hans-Joachim, Dr., W-5090 Leverkusen (DE); Lürssen, Klaus, Dr., W-5060 Bergisch Gladbach 2 (DE); Schmidt, Robert R., W-5060 Bergisch Gladbach 2 (DE); Vosswinkel, Renate, Dr., W-5067 Kürten-Bechen (DE)

(57) **Zusammenfassung**

Die Erfindung betrifft neue N-Aryl-Stickstoffheterocyclen der allgemeinen Formel (I) 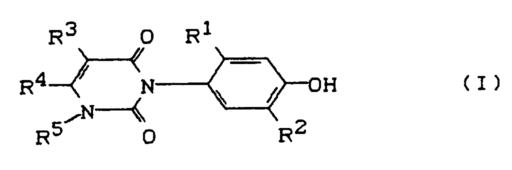
in welcher
- R¹: für Wasserstoff oder Halogen steht,
- R²: für einen der Reste 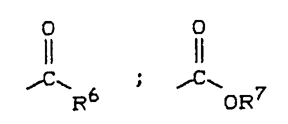 oder 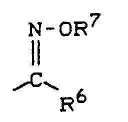 steht,
- R³: für Wasserstoff steht und
- R⁴: für Alkyl oder Halogenalkyl steht oder
- R³ und R⁴: gemeinsam für einen zweifach verknüpften Alkandiylrest stehen und
- R⁵: für einen jeweils geradkettigen oder verzweigten Rest der Reihe Alkyl, Alkenyl, Alkinyl, Halogenalkyl oder Cycloalkyl steht,
wobei
- R⁶: für Wasserstoff oder Alkyl steht und
- R⁷: für einen jeweils gegebenenfalls substituierten Rest der Reihe Alkyl, Alkenyl oder Cycloalkyl steht,
mehrere Verfahren zu ihrer Herstellung und ihre Verwendung als Herbizide.

## Beschreibung

Die Erfindung betrifft neue N-Aryl-Stickstoffheterocyclen, mehrere Verfahren zu ihrer Herstellung und ihre Verwendung als Herbizide.

Es ist bekannt, daß bestimmte N-Aryl-Stickstoffheterocyclen wie beispielsweise die Verbindung 1-(4-Cyano-2,5-difluorphenyl)-3-(1-methylethyliden)-pyrrolidin-2,-5-dion herbizide Eigenschaften besitzen (vgl. z.B. DE 38 35 168). Weitere N-Aryl-Stickstoffheterocyclen werden in DE 36 43 748, JP 10 52 755, GB 20 71 100 und DE 38 39 480 beschrieben.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden nun neue N-Aryl-Stickstoffheterocyclen der allgemeinen Formel (I), 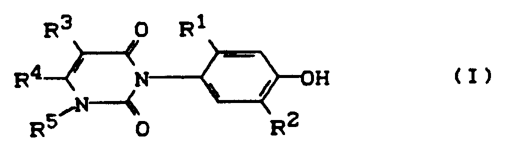
in welcher
- R¹: für Wasserstoff oder Halogon steht,
- R²: für einen der Reste 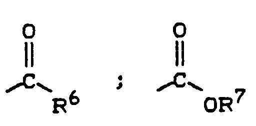 oder 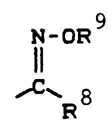 steht,
- R³: für Wasserstoff steht und
- R⁴: für Alkyl oder Halogenalkyl steht oder
- R³ und R⁴: gemeinsam für einen zweifach verknüpften Alkandiylrest stehen und
- R⁵: für einen jeweils geradkettigen oder verzweigten Rest der Reihe Alkyl, Alkenyl, Alkinyl, Halogenalkyl oder Cycloalkyl steht,
wobei
- R⁶: für Wasserstoff oder Alkyl steht
- R⁷: für einen jeweils gegebenenfalls substituierten Rest der Reihe Alkyl, Alkenyl oder Cycloalkyl steht,
- R⁸: für Wasserstoff oder Alkyl steht und
- R⁹: für einen jeweils gegebenenfalls substituierten Rest der Reihe Alkyl, Alkenyl oder Cycloalkyl steht,
gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen N-Aryl-Stickstoffheterocyclen der allgemeinen Formel (I), 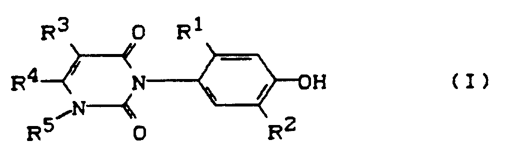
in welcher
- R¹: für Wasserstoff oder Halogen steht,
- R²: für einen der Reste 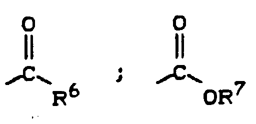 oder 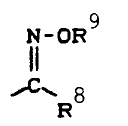 steht,
- R³: für Wasserstoff steht und
- R⁴: für Alkyl oder Halogenalkyl steht oder
- R³ und R⁴: gemeinsam für einen zweifach verknüpften Alkandiylrest stehen und
- R⁵: für einen jeweils geradkettigen oder verzweigten Rest der Reihe Alkyl, Alkenyl, Alkinyl, Halogenalkyl oder Cycloalkyl steht,
wobei
- R⁶: für Wasserstoff oder Alkyl steht
- R⁷: für einen jeweils gegebenenfalls substituierten Rest der Reihe Alkyl, Alkenyl oder Cycloalkyl steht,
- R⁸: für Wasserstoff oder Alkyl steht und
- R⁹: für einen jeweils gegebenenfalls substituierten Rest der Reihe Alkyl, Alkenyl oder Cycloalkyl steht
erhält, wenn man
a) Isocyanate der Formel (II), 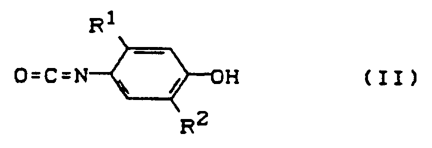 in welcher
   - R¹ und R²: die oben angegebenen Bedeutungen haben,
   mit β-Enaminoestern der Formel (III), 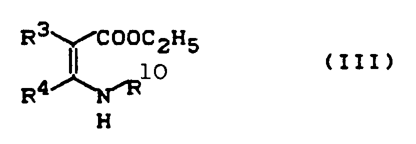 in welcher
   - R³ und R⁴: die oben angegebenen Bedeutungen haben
   und
   - R¹⁰: für Wasserstoff, oder einen jeweils geradkettigen oder verzweigten Rest der Reihe Alkyl, Alkenyl, Alkinyl, Halogenalkyl oder Cycloalkyl steht,
   gegebenenfalls in Gegenwart eines Verdünnungsmittels und gegebenenfalls in Gegenwart eines Reaktionshilfsmittels umsetzt und gegebenenfalls anschließend
b) die so erhältlichen N-Aryl-Stickstoffheterocyclen der Formel (IV), 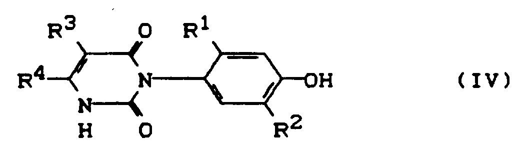 in welcher
   - R¹, R², R³ und R⁴: die oben angegebenen Bedeutungen haben,
   mit Alkylierungsmitteln der Formel (V),

   R⁵-E (V)

   in welcher
   - R⁵: die oben angegebene Bedeutung hat und
   - E: für eine elektronenanziehende Abgangsgruppe steht,
   gegebenenfalls in Gegenwart eines Verdünnungsmittels und gegebenenfalls in Gegenwart eines Reaktionshilfsmittels umsetzt oder wenn man
c) die mit Hilfe der erfindungsgemäßen Verfahren (a) oder (b) erhältlichen N-Aryl-Stickstoffheterocyclen der Formel (Ia), 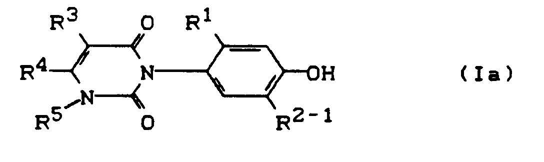 in welcher
   - R¹, R³, R⁴ und R⁵: die oben angegebenen Bedeutungen haben und
   - R²⁻¹: für einen Alkoxycarbonylrest steht,
   zunächst mit Säure gegebenenfalls in Gegenwart eines Verdünnungsmittels an der Estergruppe im Arylteil verseift und in einer anschließenden 2. Stufe die so erhältlichen N-Aryl-Stickstoffheterocyclen der Formel (VI), 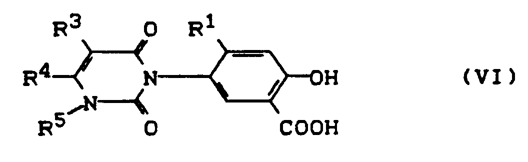 in welcher
   - R¹, R³, R⁴ und R⁵: die oben angegebenen Bedeutungen haben,
   mit Alkoholen der Formel (VII),

   R⁷-OH (VII)

   in welcher
   - R⁷: die oben angegebene Bedeutung hat,
   gegebenenfalls in Gegenwart eines Verdünnungsmittels und gegebenenfalls in Gegenwart eines Reaktionshilfsmittels verestert.

Schließlich wurde gefunden, daß die neuen N-Aryl-Stickstoffheterocyclen der allgemeinen Formel (I) herbizide Eigenschaften besitzen.

Überraschenderweise zeigen die erfindungsgemäßen N-Aryl-Stickstoffheterocyclen der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten N-Aryl-Stickstoffheterocyclen, wie beispielsweise die Verbindung 1-(4-Cyano-2,5-difluorphenyl)-3-(1-methylethyliden)-pyrrolidin-2,5-dion, welche chemisch und wirkungsmäßig naheliegende Verbindungen sind.

Die erfindungsgemäßen N-Aryl-Stickstoffheterocyclen sind durch die Formel (I) allgemein definiert. Bevorzugt sind Verbindungen der Formel (I), bei welchen
- R¹: für Wasserstoff, Fluor, Chlor, Brom oder Iod steht,
- R²: für einen der Reste 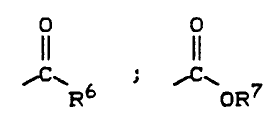 oder 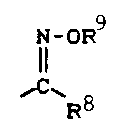 steht,
- R³: für Wasserstoff steht und
- R⁴: für jeweils geradkettiges oder verzweigtes Alkyl mit 1 bis 6 Kohlenstoffatomen oder Halogenalkyl mit 1 bis 6 Kohlenstoffatomen und 1 bis 13 gleichen oder verschiedenen Halogenatomen steht oder
- R³ und R⁴: gemeinsam für einen zweifach verknüpften, geradkettigen oder verzweigten Alkandiylrest mit 3 bis 10 Kohlenstoffatomen stehen und
- R⁵: für ein jeweils geradkettigen oder verzweigten Rest der Reihe Alkyl mit 1 bis 6 Kohlenstoffatomen, Halogenalkyl mit 1 bis 6 Kohlenstoffatomen und 1 bis 13 gleichen oder verschiedenen Halogenatomen, Alkenyl mit 2 bis 6 Kohlenstoffatomen, Alkinyl mit 2 bis 6 Kohlenstoffatomen oder für Cycloalkyl mit 3 bis 8 Kohlenstoffatomen steht,
wobei
- R⁶: für Wasserstoff oder für geradkettiges oder verzweigtes Alkyl mit 1 bis 6 Kohlenstoffatomen steht,
- R⁷: für einen jeweils geradkettigen oder verzweigten Rest der Reihe Alkyl mit 1 bis 12 Kohlenstoffatomen, Halogenalkyl mit 1 bis 6 Kohlenstoffatomen und 1 bis 13 gleichen oder verschiedenen Halogenatomen, Alkoxyalkyl, Alkoxyalkoxyalkyl oder Alkoxycarbonylalkyl mit jeweils 1 bis 6 Kohlenstoffatomen in den einzelnen Alkylteilen, Alkenyl mit 2 bis 6 Kohlenstoffatomen oder für gegebenenfalls einfach oder mehrfach, gleich oder verschieden durch geradkettiges oder verzweigtes Alkyl mit 1 bis 4 Kohlenstoffatomen substituiertes Cycloalkyl mit 3 bis 8 Kohlenstoffatomen steht,
- R⁸: für Wasserstoff oder für geradkettiges oder verzweigtes Alkyl mit 1 bis 6 Kohlenstoffatomen steht und
- R⁹: für einen jeweils geradkettigen oder verzweigten Rest der Reihe Alkyl mit 1 bis 12 Kohlenstoffatomen, Halogenalkyl mit 1 bis 6 Kohlenstoffatomen und 1 bis 13 gleichen oder verschiedenen Halogenatomen, Alkoxyalkyl, Alkoxyalkoxyalkyl oder Alkoxycarbonylalkyl mit jeweils 1 bis 6 Kohlenstoffatomen in den einzelnen Alkylteilen, Alkenyl mit 2 bis 6 Kohlenstoffatomen oder für gegebenenfalls einfach oder mehrfach, gleich oder verschieden durch geradkettiges oder verzweigtes Alkyl mit 1 bis 4 Kohlenstoffatomen Substituiertes Cycloalkyl mit 3 bis 8 Kohlenstoffatomen steht.

Besonders bevorzugt sind Verbindungen der Formel (I), bei welchen
- R¹: für Wasserstoff, Fluor, Chlor oder Brom steht,
- R²: für einen der Reste 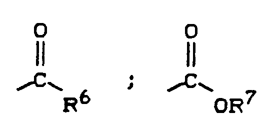 oder 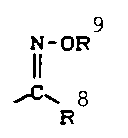 steht,
- R³: für Wasserstoff steht und
- R⁴: für jeweils geradkettiges oder verzweigtes Alkyl mit 1 bis 4 Kohlenstoffatomen oder Halogenalkyl mit 1 bis 4 Kohlenstoffatomen und 1 bis 9 gleichen oder verschiedenen Halogenatomen steht oder
- R³ und R⁴: gemeinsam für einen zweifach verknüpften, geradkettigen oder verzweigten Alkandiylrest mit 3 bis 8 Kohlenstoffatomen stehen und
- R⁵: für einen jeweils geradkettigen oder verzweigten Rest der Reihe Alkyl mit 1 bis 4 Kohlenstoffatomen, Halogenalkyl mit 1 bis 4 Kohlenstoffatomen und 1 bis 9 gleichen oder verschiedenen Halogenatomen, Alkenyl mit 2 bis 5 Kohlenstoffatomen, Alkinyl mit 2 bis 5 Kohlenstoffatomen oder für Cycloalkyl mit 3 bis 7 Kohlenstoffatomen steht,
wobei
- R⁶: für Wasserstoff oder für geradkettiges oder verzweigtes Alkyl mit 1 bis 3 Kohlenstoffatomen steht,
- R⁷: für einen jeweils geradkettigen oder verzweigten Rest der Reihe Alkyl mit 1 bis 8 Kohlenstoffatomen, Halogenalkyl mit 1 bis 4 Kohlenstoffatomen und 1 bis 9 gleichen oder verschiedenen Halogenatomen, Alkoxyalkyl, Alkoxyalkoxyalkyl oder Alkoxycarbonylalkyl mit jeweils 1 bis 4 Kohlenstoffatomen in den einzelnen Alkylteilen, Alkenyl mit 2 bis 4 Kohlenstoffatomen oder für gegebenenfalls einfach bis dreifach, gleich oder verschieden durch geradkettiges oder verzweigtes Alkyl mit 1 bis 3 Kohlenstoffatomen substituiertes Cycloalkyl mit 3 bis 7 Kohlenstoffatomen steht,
- R⁸: für Wasserstoff oder für geradkettiges oder verzweigtes Alkyl mit 1 bis 3 Kohlenstoffatomen steht und
- R⁹: für einen jeweils geradkettigen oder verzweigten Rest der Reihe Alkyl mit 1 bis 8 Kohlenstoffatomen, Halogenalkyl mit 1 bis 4 Kohlenstoffatomen und 1 bis 9 gleichen oder verschiedenen Halogenatomen, Alkoxyalkyl, Alkoxyalkoxyalkyl oder Alkoxycarbonylalkyl mit jeweils 1 bis 4 Kohlenstoffatomen in den einzelnen Alkylteilen, Alkenyl mit 2 bis 4 Kohlenstoffatomen oder für gegebenenfalls einfach bis dreifach, gleich oder verschieden durch geradkettiges oder verzweigtes Alkyl mit 1 bis 3 Kohlenstoffatomen substituiertes Cycloalkyl mit 3 bis 7 Kohlenstoffotomen steht.

Ganz besonders bevorzugt sind Verbindungen der Formel (I), bei welchen
- R¹: für Wasserstoff oder Fluor steht,
- R²: für einen der Reste 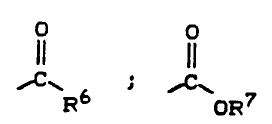 oder 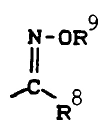 steht,
- R³: für Wasserstoff steht und
- R⁴: für jeweils geradkettiges oder verzweigtes Alkyl mit 1 bis 3 Kohlenstoffatomen oder Halogenalkyl mit 1 bis 2 Kohlenstoffatomen und 1 bis 5 gleichen oder verschiedenen Halogenatomen steht oder
- R³ und R⁴: gemeinsam für einen zweifach verknüpften, geradkettigen oder verzweigten Alkandiylrest mit 3 bis 6 Kohlenstoffatomen stehen und
- R⁵: für einen jeweils geradkettigen oder verzweigten Rest der Reihe Alkyl mit 1 bis 3 Kohlenstoffatomen, Halogenalkyl mit 1 bis 2 Kohlenstoffatomen und 1 bis 5 gleichen oder verschiedenen Halogenatomen, Alkenyl mit 2 bis 4 Kohlenstoffatomen, Alkinyl mit 2 bis 4 Kohlenstoffatomen oder für Cyclohexyl steht,
wobei
- R⁶: für Wasserstoff oder für geradkettiges oder verzweigtes Alkyl mit 1 bis 3 Kohlenstoffatomen steht,
- R⁷: für einen jeweils geradkettigen oder verzweigten Rest der Reihe Alkyl mit 1 bis 6 Kohlenstoffatomen, Halogenalkyl mit 1 bis 3 Kohlenstoffatomen und 1 bis 7 gleichen oder verschiedenen Halogenatomen, Alkoxyalkyl, Alkoxyalkoxyalkyl oder Alkoxycarbonylalkyl mit jeweils 1 bis 3 Kohlenstoffatomen in den einzelnen Alkylteilen, Alkenyl mit 2 bis 3 Kohlenstoffatomen oder für gegebenenfalls einfach oder zweifach durch Methyl substituiertes Cyclopropyl, Cyclopentyl oder Cyclohexyl steht,
- R⁸: für Wasserstoff oder für geradkettiges oder verzweigtes Alkyl mit 1 bis 3 Kohlenstoffatomen steht und
- R⁹: für einen jeweils geradkettigen oder verzweigten Rest der Reihe Alkyl mit 1 bis 6 Kohlenstoffatomen, Halogenalkyl mit 1 bis 3 Kohlenstoffatomen und 1 bis 7 gleichen oder verschiedenen Halogenatomen, Alkoxyalkyl, Alkoxyalkoxyalkyl oder Alkoxycarbonylalkyl mit jeweils 1 bis 3 Kohlenstoffatomen in den einzelnen Alkylteilen, Alkenyl mit 2 bis 3 Kohlenstoffatomen oder für gegebenenfalls einfach oder zweifach durch Methyl substituiertes Cyclopropyl, Cyclopentyl oder Cyclohexyl steht.

Verwendet man beispielsweise 2-Fluor-4-hydroxy-5-methoxycarbonyl-phenylisocyanat und 3-(N-Methylamino)-4,4,4-trifluor-crotonsäuremethylester als Ausgangsstoffe, so läßt sich der Reaktionsablauf des erfindungsgemäßen Verfahrens (a) durch das folgende Formelschema darstellen: 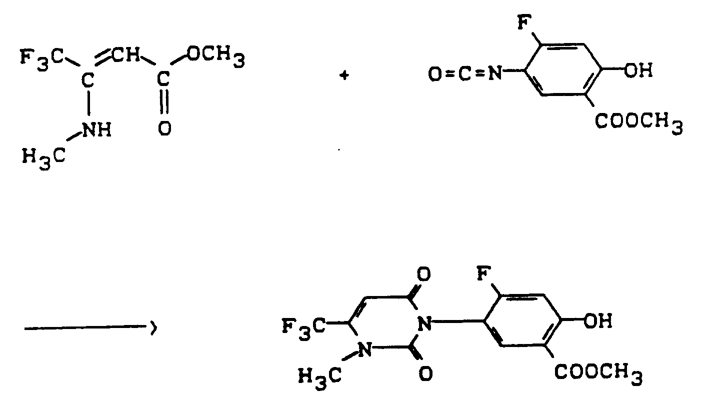
Verwendet man beispielsweise 3-(4-Hydroxy-3-methoxycarbonyl-phenyl)-6-trifluormethyl-1,2,3,4-tetrahydro-1H-pyrimidin-2,4-dion und Methyliodid als Ausgangsstoffe, so läßt sich der Reaktionsablauf des erfindungsgemäßen Verfahrens (b) durch das folgende Formelschema darstellen: 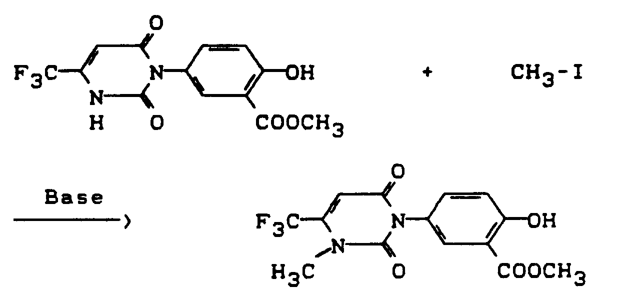
Verwendet man beispielsweise 3-(4-Hydroxy-3-methoxycarbonyl-phenyl)-1-methyl-6-trifluormethyl-1,2,3,4-tetrahydro-pyrimidin-2,4-dion und Cyclohexanol als Ausgangsstoffe, so läßt sich der Reaktionsablauf des erfindungsgemäßen Verfahrens (c) durch das folgende Formelschema darstellen: 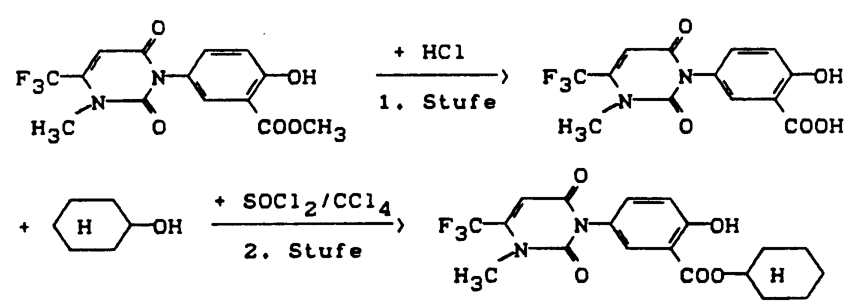
Die zur Durchführung des erfindungsgemäßen Verfahrens (a) als Ausgangsstoffe benötigten Isocyanate sind durch die Formel (II) allgemein definiert, In dieser Formel (II) stehen R¹ und R² vorzugsweise für diejenigen Reste, die bereits im Zusammenhang mit der Beschreibung der erfindungsgemäßen Stoffe der Formel (I) als bevorzugt für diese Substituenten genannt wurden, Die Isocyanate der Formel (II) sind noch nicht bekannt und ebenfalls Gegenstand der Erfindung. Man erhält sie, wenn man Anilin-Derivate der Formel (VIII), 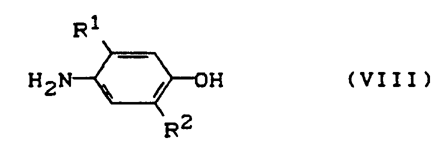
in welcher
- R¹ und R²: die oben angegebene Bedeutung haben,
mit Chlorameisensäuretrichlormethylester (Diphosgen) gegebenenfalls in Gegenwart eines Verdünnungsmittels wie beispielsweise Essigsäureethylester bei Temperaturen zwischen 50°C und 150°C umsetzt.

Anilin-Derivate der Formel (VIII) sind bekannt oder erhältlich in Analogie zu bekannten Verfahren (vgl. z.B. EP 291 159; EP 275 221; EP 253 788; EP 262 618; DE 36 38 364; PCT WO 8603194; J. Org. Chem, 54, 3740-3744 [1989]).

Die zur Durchführung das erfindungsgemäßen Verfahrens (a) als Ausgangsstoffe benötigten β-Enaminoester sind durch die Formel (III) allgemein definiert. In dieser Formel (III) stehen R³ und R⁴ vorzugsweise für diejenigen Raste, die bereits im Zusammenhang mit dar Beschreibung dar erfindungsgemäßen Stoffe dar Formel (I) als bevorzugt für diese Substituenten genannt wurden.

R¹⁰steht vorzugsweise für Wasserstoff oder für einen jeweils geradkettigen oder verzweigten Rest der Reihe Alkyl mit 1 bis 6 Kohlanstoffatomen, Halogenalkyl mit 1 bis 6 Kohlenstoffatomen und 1 bis 13 gleichen oder verschiedenen Halogenatomen, Alkenyl mit 2 bis 6 Kohlenstoffatomen, Alkinyl mit 2 bis 6 Kohlenstoffatoman oder für Cycloalkyl mit 3 bis 8 Kohlenstoffatomen.

R¹⁰steht besonders bevorzugt für einen jeweils geradkettigen oder verzweigtan Rast der Reihe Alkyl mit 1 bis 4 Kohlenstoffatomen, Halogenalkyl mit 1 bis 4 Kohlenstoffatomen und 1 bis 9 gleichen oder verschiedenen Halogenatomen, Alkenyl mit 2 bis 5 Kohlenstoffatoman, Alkinyl mit 2 bis 5 Kohlanstoffatomen oder für Cycloalkyl mit 3 bis 7 Kohlanstoffatomen.

Die β-Enaminoester der Formel (III) sind bekannt oder er- hältlich in Analogie zu bekannten Verfahren (vgl. z.B. PCT WO 9107392; EP 304 409; J. Heterocycl. Chem. 9, 513-522 [1972]; EP 295 233).

Die zur Durchführung das erfindungsgemäßen Verfahrens (b) als Ausgangsstoffe benötigten N-Aryl-Stickstoffheterocyclen sind durch die Formel (IV) allgemein definiert. In dieser Formel (IV) stehen R¹, R², R³ und R⁴ vorzugsweise für diejenigen Reste, die bereits im Zusammenhang mit der Beschreibung der erfindungsgemäßen Stoffe der Formel (I) als bevorzugt für diese Substituenten genannt wurden.

Die N-Aryl-Stickstoffheterocyclen der Formel (IV) sind noch nicht bekannt und ebenfalls Gegenstand der Erfindung. Man erhalt sie mit Hilfe des erfindungsgemäßen Verfahrens (a).

Die zur Durchführung des erfindungsgemäßen Verfahrens (b) weiterhin als Ausgangsstoffe benötigten Alkylierungsmittel sind durch die Formel (V) allgemein definiert. In dieser Formel (V) steht R⁵ vorzugsweise für diejenigen Reste, die bereits im Zusammenhang mit der Beschreibung der erfindungsgemäßen Stoffe der Formel (I) als bevorzugt für diese Substituenten genannt wurden.

E steht für einen bei Alkylierungsmitteln üblichen Abgangsrest, vorzugsweise für Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

Die Alkylierungsmittel der Formel (V) sind allgemein bekannte Verbindungen dar organischen Chemie.

Die zur Durchführung des erfindungsgemäßan Verfahrens (c) als Ausgangsstoffe benötigten N-Aryl-Stickstoffheterocyclen sind durch die Formel (Ia) allgemein definiert. In dieser Formel (Ia) stehen R¹, R³, R⁴ und R⁵ vorzugsweise für diejenigen Reste, die bereits im Zusammenhang mit der Beschreibung der erfindungsgemäßen Stoffe dar Formel (I) als bevorzugt für diese Substituenten genannt wurden.
R²⁻¹ steht vorzugsweise für einen geradkettigen oder verzweigten Alkoxycarbonylrest mit 1 bis 4 Kohlenstoffatomen im Alkoxyteil, insbesondere für einen Methoxycarbonylrest oder für einen Ethoxycarbonylrest.
Die N-Aryl-Stickstoffheterocyclen der Formel (Ia) sind erfindungsgemäße Verbindungen und erhältlich mit Hilfe der erfindungsgemäßen Verfahren (a) und/oder (b).

Die bei der Durchführung des erfindungsgemäßen Verfahrens (c) als Zwischenprodukte auftretenden N-Aryl-Stickstoffheterocyclen sind durch die Formel (VI) allgemein definiert. In dieser Formel (VI) stehen R¹, R³, R⁴ und R⁵ vorzugsweise für diejenigen Reste, die bereits im Zusammenhang mit der Beschreibung der erfindungsgemäßen Stoffe der Formel (I) als bevorzugt für diese Substituenten genannt wurden.
Die N-Aryl-Stickstoffheterocyclen der Formel (VI) sind noch nicht bekannt und ebenfalls Gegenstand der Erfindung. Man erhält sie mit Hilfe das erfindungsgemäßen Verfahrens (c).

Mie zur Durchführung das erfindungsgemäßen Verfahrens (c) weiterhin als Ausgangsstoffe benötigten Alkohole sind durch die Formel (VII) allgemein definiert. In dieser Formel (VII) steht R⁷ vorzugsweise für diejenigen Reste, die bereits im Zusammenhang mit dar Beschreibung der erfindungsgemäßen Stoffe der Formel (I) als bevorzugt für diese Substituenten genannt wurden.
Die Alkohole der Formel (VII) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester oder Sulfoxide wie Dimethylsulfoxid.

Das erfindungsgemäße Verfahren (a) wird vorzugsweise in Gegenwart eines geeigneten Reaktionshilfsmittels durchgeführt. Als solche kommen alle üblichen anorganischen oder organischen Basen in Frage. Hierzu gehören beispielsweise Erdalkali-oder Alkalimetallhydroxide wie Natriumhydroxid, Calciumhydroxid, Kaliumhydroxid oder auch Ammoniumhydroxid, Alkalimetallcarbonate wie Natriumcarbonat, Kaliumcarbonat, Kaliumhydrogencarbonat, Natriumhydrogencarbonat oder Ammoniumcarbonat, Alkali- oder Erdalkalimetallacetate wie Natriumacetat, Kaliumacetat, Calciumacatat oder Ammoniumacetat sowie tertiäre Amine wie Trimethylamin, Triethylamin Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen -70°C und +50°C, vorzugsweise bei Temperaturen zwischen -40°C und +20°C.

Zur Durchführung des erfindungsgemäßen Verfahrens (a) setzt man pro Mol Isocyanat der Formel (II) im allgemeinen 1.0 bis 1,5 Mol, vorzugsweise 1.0 bis 1,2 Mol an Aminoester der Formel (III) und gegebenenfalls 1.0 bis 5,0 Mol, vorzugsweise 1.0 bis 2,5 Mol Base als Reaktionshilfsmittel ein.
Die Reaktionsdurchführung, Aufarbeitung und Isolierung der Reaktionsprodukte erfolgt nach allgemein üblichen Methoden (vgl. hierzu die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester oder Sulfoxide wie Dimethylsulfoxid.

Das erfindungsgemäße Verfahren (b) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-G₁₃/C₁₅-alkylammoniumchlorid, Dibenzyl-dimethyl-ammoniummethylsulfat, Dimethyl-C₁₂/C₁₄-alkyl-benzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und 150°C, vorzugsweise bei Temperaturen zwischen 0°C und 20°C.

Zur Durchführung des erfindungsgemäßen Verfahrens (b) setzt man pro Mol N-Aryl-Stickstoffheterocyclus der Formel (IV) im allgemeinen 1.0 bis 2,5 Mol, vorzugsweise 1.0 bis 1,5 Mol Alkylierungsmittel der Formel (V) und gegebenenfalls 1.0 bis 2,5 Mol, vorzugsweise 1.0 bis 1,5 Mol Base als Reaktionshilfsmittel und gegebenenfalls 0,001 bis 2,0 Mol, vorzugsweise 0,01 bis 1,0 Mol Phasentransferkatalysator ein.
Die Reaktionsdurchführung, Aufarbeitung und Isolierung der Reaktionsprodukte erfolgt nach allgemein üblichen Methoden (vgl. hierzu die Herstellungsbeispiele).

Zur Durchführung der ersten Stufe des erfindungsgemäßen Verfahrens (c) kommen als esterspaltende Reagenzien alle üblichen anorganischen und organischen Säuren infrage.

Vorzugsweise verwendet man wäßrige Mineralsäuren, wie beispielsweise Salzsäure oder Schwefelsäure.

Als Verdünnungsmittel zur Durchführung der ersten Stufe des erfindungsgemäßen Verfahrens (c) kommen alle üblichen organischen oder anorganischen Lösungsmittel infrage. Vorzugsweise verwendet man polare mit Wasser mischbare organische Lösungsmittel, insbesondere Alkohole, wie Methanol, Ethanol, Propanol oder Butanol, deren Gemische mit Wasser oder reines Wasser als Lösungsmittel.

Die Reaktionstemperaturan können bei der Durchführung der ersten Stufe des erfindungsgemäßen Verfahrens (c) in einem größeren Bereich variiert werden. Im allgemeinen arbeitet man bei Temperaturen zwischen -20°C und 120°C, vorzugsweise bei Temperaturen zwischen 0°C und 80°C.

Zur Durchführung dar ersten Stufe des erfindungsgemäßen Verfahrens (c) setzt man pro Mol N-Aryl-Stickstoffheterocyclus der Formel (Ia) im allgemeinen 1,0 bis 10,0 Mol, vorzugsweise 1,0 bis 5,0 Mol wäßrige Mineralsäure ein. Die Reaktionsdurchführung, Aufarbeitung und Isolierung der Reaktionsprodukte erfolgt nach allgemein bekannten Verfahren.

Als Verdünnungsmittel zur Durchführung der zweiten Stufe des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid. Es ist auch möglich, einen entsprechenden Überschuß an eingesetztem Alkohol der Formel (VII) als Reaktionspartner und gleichzeitig als Verdünnungsmittel zu verwenden.

Die zweite Stufe des erfindungsgemäßen Verfahrens (c) kann gegebenenfalls in Gegenwart eines geeigneten Reaktionshilfsmittel durchgeführt werden, Als solche kommen alle üblichen anorganischen oder organischen Basen in Frage. Hierzu gehören beispielsweise Erdalkali- oder Alkalimetallhydroxide wie Natriumhydroxid, Calciumhydroxid, Kaliumhydroxid oder auch Ammoniumhydroxid, Alkalimetallcarbonate wie Natriumcarbonat, Kaliumcarbonat, Kaliumhydrogencarbonat, Natriumhydrogencarbonat oder Ammoniumcarbonat, Alkali-der Erdalkalimetallacetate wie Natriumacetat, Kaliumacetat, Calciumacetat oder Ammoniumacetat sowie tertiäre Amine wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der zweiten Stufe des erfindungsgemäßen Verfahrens (c) in einem größeren Bereich variiert werden, Im allgemeinen arbeitet man bei Temperaturen zwischen -20°C und +150°C, vorzugsweise bei Temperaturen zwischen 0°C und 120°C.

Zur Durchführung der zweiten Stufe des erfindungsgemäßen Verfahrens (c) setzt man pro Mol N-Aryl-Stickstoffheterocyclus der Formel (VI) im allgemeinen 1,0 bis 10,0 Mol, vorzugsweise 1,0 bis 5,0 Mol Alkohol dar Formel (VII) und gegebenenfalls 0,001 bis 2,0 Mol, vorzugsweise 0,001 bis 1,0 Mol Reaktionshilfsmittel ein.

Die Reinigung der Endprodukte der Formel (I) erfolgt mit Hilfe üblicher Verfahren, beispielsweise durch Säulenchromatographie oder durch Umkristallisieren. Die Charakterisierung erfolgt mit Hilfe des Schmelzpunktes oder bei nicht kristallisierenden Verbindungen mit Hilfe dar Protonen-Kernresonanzspektroskopie (¹H-NMR).

Die erfindungsgemäßen Wirkstoffe können als Defoliants, Desiccants, Krautabtötungsmittel und insbesondere als Unkrautvernichtungsmittel verwendet werden, Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopacurus, Apera.

Monokotyle Kulturen der Gattungen: Oryza, Zea, Triticum, Hordaum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beeranfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern in monokotylen und dikotylen Kulturen wie beispielsweise Weizen oder Baumwolle einsetzen.

Daneben greifen die erfindungsgemäßen Wirkstoffe auch in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daS ein Wirkstoff auch mehrere verschiedenartige Wirkungen auf Pflanzen ausüben kann. Die Wirkungen der Stoffe hängan im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächanaktiven Mitteln, also Emulgiermitteln und/oder Dispergiermitteln und/oder schaumerzeugenden Mitteln.

Im Falle dar Benutzung von Wasser als Streckmittal können z.B. auch organische Lösungsmittel als Hilfslösungsmittel verwendet werden, Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalan, Maiskolban und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fattalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein,

Es können Farbstoffe wie anorganische Pigmente, z.B, Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phanmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuronmethyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmathylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungizidan, Insektizidan, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie könnan auch vor der Saat in den Boden eingearbeitet werden.

Die angewandte Wirkstoffmenge kann in einem größeren Bereich schwanken, Sie hängt im wesentlichen von der Art des gewünschten Effektes ab, Im allgemeinen liegen die Aufwandmengen zwischen 1 g und 10 kg Wirkstoff pro Hektar Bodenfläche, vorzugsweise zwischen 5 g und 5 kg pro ha,

Die Herstellung und die Verwendung dar erfindungsgemäßen Wirkstoffe geht aus den nachfolgenden Beispielen hervor.

### Herstellungsbeispiele:

### Beispiel 1:

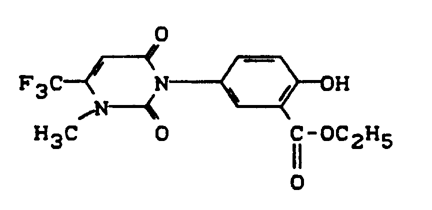

### (Verfahren (b))

Zu 2,1 g (0,006 Mol) 3-(4-Hydroxy-3-ethoxycarbonyl-phenyl)-6-trifluormethyl-1,2,3,4-tetrahydro-1H-pyrimidin-2,4-dion in 50 ml Dimethylsulfoxid gibt man nacheinander 0,8 g (0,006 Mol) wasserfreies Kaliumcarbonat und 1 g (0,006 Mol) Methyliodid und rührt 16 Stunden bei Raumtemperatur. Zur Aufarbeitung wird die Reaktionsmischung in Wasser gegeben, mit Salzsaure angesäuert und mit Dichlormethan extrahiert. Die organische Phase wird über Magnesiumsulfat getrocknet, im Vakuum eingeengt und der Rückstand durch Chromatographie an Kieselgel (Laufmittel: Dichlormethan) gereinigt.

Man erhält 1 g (46,5% der Theorie) 3-(4-Hydroxy-3-ethoxycarbonyl-phenyl)-1-methyl-6-trifluormethyl-1,2,3,4-tetrahydro-pyrimidin-2,4-dion vom Schmelzpunkt 65°C.

### Herstellung der Ausgangsverbindung

### Beispiel IV-1:

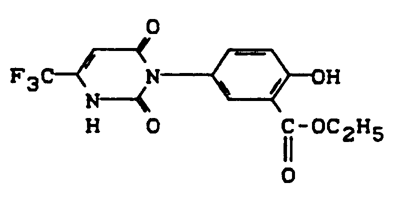

### (Verfahren (a))

Zu einer bei 0°C hergestellten Suspension von 3,0 g (0,1 Mol) Natriumhydrid (80%ig in Paraffinöl) in 50 ml Dimethylformamid gibt man bei -30°C tropfenweise unter Rühren nacheinander 9,15 g (0,05 Mol) 3-Amino-4,4,4-trifluor-crotonsäureethylester (vgl. z.B. J. Heterocycl. Chem. 9,513 [1972]) in 20 ml Dimethylformamid und 10,35 g (0,05 Mol) 4-Hydroxy-3-ethoxycarbonyl-phenylisocyanat in 100 ml Toluol und rührt nach beendeter Zugabe 6 Stunden bei -30°C. Zur Aufarbeitung wird die Reaktionsmischung in Wasser gegeben, mit Salzsäure angesäuert und mit Essigaster extrahiert. Die organische Phase wird über Magnesiumsulfat getrocknet und im Vakuum vom Lösungsmittal befreit.

Man erhält 10,3 g (60% der Theorie) 3-(4-Hydroxy-3-ethoxycarbonylphenyl)-6-trifluormethyl-1,2,3,4-tetrahydro-1H-pyrimidin-2,4-dion vom Schmelzpunkt 157-59°C.

### Beispiel II-1:

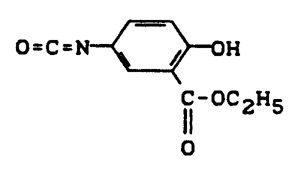

Zu 9,05 g (0,05 Mol) 5-Amino-2-hydroxy-benzoesäureethylester (vgl. z.B. EP 291 159) in 150 ml Essigester gibt man bei 70°C tropfenweise unter Rühren 9,9 g (0,05 Mol) Chlorameisensäuretrichlormethylester und erhitzt für zwei Stunden auf Rückflußtemperatur. Zur Aufarbeitung wird die Reaktionsmischung im Vakuum eingeengt und der Rückstand in 100 ml wasserfreiem Toluol aufgenommen.

Die so erhältliche Lösung von 4-Hydroxy-3-ethoxycarbonyl-phenylisocyanat wird ohne Isolierung direkt weiter umgesetzt.
IR (Nacl): ν = 2272 cm⁻¹
In entsprechender Weise und gemäß den allgemeinen Angaben zur Herstellung erhält man die folgenden N-Aryl-Stickstoffheterocyclen der allgemeinen Formel (I):

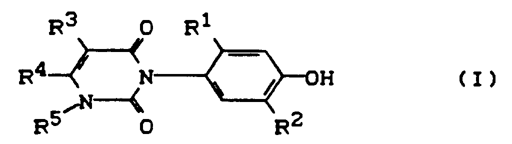

| Bsp.-Nr. | R¹ | R² | R³ | R⁴ | R⁵ | physikalische Eigenschaften |
|---|---|---|---|---|---|---|
| 2 | H | -C(O)-O-iC₃H₇ | H | CF₃ | CH₃ | 3.39 (s; 3H) |
| 3 | H | -C(O)-O-CH₃ | H | CF₃ | CH₃ | Fp 185°C |
| 4 | H | -C(O)-iC₃H₇ | H | CF₃ | CH₃ | ? |
| IV-2 | H | -C(O)-O-CH₃ | H | CF₃ | H | Fp 201°C |

| | | | | | | |
|---|---|---|---|---|---|---|
| *) Die ¹H-NMR-Spektren wurden in Deuterochloroform (CDCl₃) mit Tetramethylsilan (TMS) als innerem Standard aufgenommen. Angegeben ist die chemische Verschiebung als δ-Wert in ppm. | | | | | | |

### Anwendungsbeispiele:

In den folgenden Anwendungsbeispielen wurde die nachstehend aufgeführte Verbindung als Vergleichssubstanz eingesetzt: 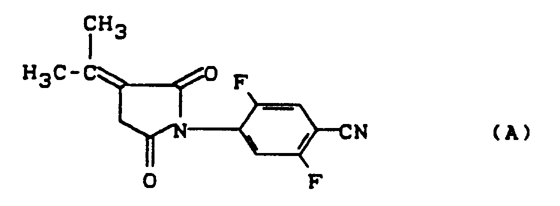
1-(4-Cyano-2,5-difluorphenyl)-3-(1-methylethyliden)-pyrrolidin-2,5-dion
(vgl. z.B. DE 38 35 168)

### Beispiel A:

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffmengen pro Flächeneinheit ausgebracht werden, Die Konzentration der Spritzbrühe wird so gewählt, daS in 2000 l Wasser pro Hektar die jeweils gewünschten Wirkstoffmengan ausgebracht werden. Nach drei Wochen wird der Schädigungsgrad der Pflanzen bonitiert in % Schädigung im Vergleich zur Entwicklung der unbehandelten Kontrolle.
Es bedeuten:
0% = keine Wirkung (wie unbehandelte Kontrolle)
100% = totale Vernichtung
Eine deutliche Überlegenheit in der Wirksamkeit bei vergleichbarer Nutzpflanzenselektivität gegenüber dem Stand der Technik zeigt in diesem Test z.B. die Verbindung gemäß Herstellungsbeispiel 1.

## Patentansprüche

1. N-Aryl-Stickstoffheterocyclen der allgemeinen Formel (I) 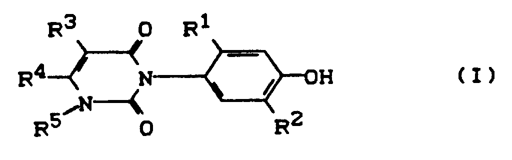 in welcher
R¹ für Wasserstoff oder Halogen steht,
R² für einen der Reste 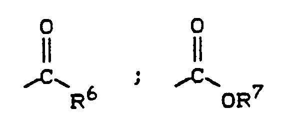 oder 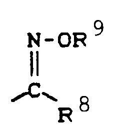 steht,
R³ für Wasserstoff steht und
R⁴ für Alkyl oder Halogenalkyl steht oder
R³ und R⁴ gemeinsam für einen zweifach verknüpften Alkandiylrest stehen und
R⁵ für einen jeweils geradkettigen oder verzweigten Rast der Reihe Alkyl, Alkenyl, Alkinyl, Halogenalkyl oder Cycloalkyl steht,
wobei
R⁶ für Wasserstoff oder Alkyl steht,
R⁷ für einen jeweils gegebenenfalls substituierten Rest der Reihe Alkyl, Alkenyl oder Cycloalkyl steht,
R⁸ für Wasserstoff oder Alkyl steht und
R⁹ für einen jeweils gegebenenfalls substituierten Rest der Reihe Alkyl, Alkenyl oder Cycloalkyl steht.

2. N-Aryl-Stickstoffheterocyclen dar allgemeinen Formel (I) gemäß Anspruch 1, dadurch gekennzeichnet, daß
R¹ für Wasserstoff, Fluor, Chlor, Brom oder Iod steht,
R² für einen der Reste 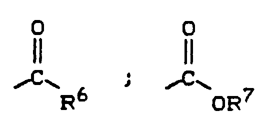 oder 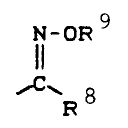 steht,
R³ für Wasserstoff steht und
R⁴ für jeweils geradkettiges oder verzweigtes Alkyl mit 1 bis 6 Kohlenstoffatomen, Halogenalkyl mit 1 bis 6 Kohlenstoffatomen und 1 bis 13 gleichen oder verschiedenen Halogenatomen steht oder
R³ und R⁴ gemeinsam für einen zweifach verknüpften, geradkettigen oder verzweigten Alkandiylrest mit 3 bis 10 Kohlenstoffatomen stehen und
R⁵ für einen jeweils geradkettigen oder verzweigtan Rast der Reihe Alkyl mit 1 bis 6 Kohlenstoffatomen Halogenalkyl mit 1 bis 6 Kohlanstoffatomen und 1 bis 13 gleichen oder verschiedenen Halogenatomen, Alkenyl mit 2 bis 6 Kohlanstoffatomen, Alkinyl mit 2 bis 6 Kohlanstoffatomen oder für Cycloalkyl mit 3 bis 8 Kohlenstoffatomen steht,
wobei
R⁶ für Wasserstoff oder für geradkettiges oder verzweigtes Alkyl mit 1 bis 6 Kohlenstoffatoman steht,
R⁷ für einen jeweils geradkettigen oder verzweigten Rest der Reihe Alkyl mit 1 bis 12 Kohlenstoffatomen, Halogenalkyl mit 1 bis 6 Kohlenstoffatomen und 1 bis 13 gleichen oder verschiedenen Halogenatomen, Alkoxyalkyl, Alkoxyalkoxyalkyl oder Alkoxycarbonylalkyl mit jeweils 1 bis 6 Kohlenstoffatomen in den einzelnen Alkylteilen, Alkenyl mit 2 bis 6 Kohlenstoffatomen oder für gegebenenfalls einfach oder mehrfach, gleich oder verschieden durch geradkettiges oder verzweigtes Alkyl mit 1 bis 4 Kohlenstoffatomen substituiertes Cycloalkyl mit 3 bis 8 Kohlenstoffatoman steht,
R⁸ für Wasserstoff oder für geradkettiges oder verzweigtes Alkyl mit 1 bis 6 Kohlenstoffatomen steht und
R⁹ für einen jeweils geradkettigen oder verzweigten Rest der Reihe Alkyl mit 1 bis 12 Kohlenstoffatomen, Halogenalkyl mit 1 bis 6 Kohlenstoffatomen und 1 bis 13 gleichen oder verschiedenen Halogenatomen, Alkoxyalkyl, Alkoxyalkoxyalkyl oder Alkoxycarbonylalkyl mit jeweils 1 bis 6 Kohlenstoffatomen in den einzelnen Alkylteilen, Alkenyl mit 2 bis 6 Kohlenstoffatomen oder für gegebenenfalls einfach oder mehrfach, gleich oder verschieden durch geradkettiges oder verzweigtes Alkyl mit 1 bis 4 Kohlenstoffatomen substituiertes Cycloalkyl mit 3 bis 8 Kohlenstoffatomen steht.

3. N-Aryl-Stickstoffheterocyclen der allgemeinen Formel (I) gemäß Anspruch 1, dadurch gekennzeichnet, daß
R¹ für Wasserstoff, Fluor, Chlor oder Brom steht,
R² für einen der Reste 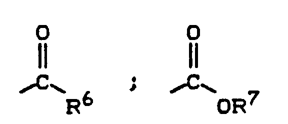 oder 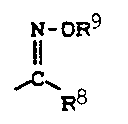 steht,
R³ für Wasserstoff steht und
R⁴ für jeweils geradkettiges oder verzweigtes Alkyl mit 1 bis 4 Kohlenstoffatomen oder Halogenalkyl mit 1 bis 4 Kohlenstoffatomen und 1 bis 9 gleichen oder verschiedenen Halogenatomen steht oder
R³ und R⁴ gemeinsam für einen zweifach verknüpften, geradkettigen oder verzweigten Alkandiylrest mit 3 bis 8 Kohlenstoffatomen stehen und
R⁵ für einen jeweils geradkettigen oder verzweigten Rest der Reihe Alkyl mit 1 bis 4 Kohlenstoffatomen, Halogenalkyl mit 1 bis 4 Kohlenstoffatomen und 1 bis 9 gleichen oder verschiedenen Halogenatomen, Alkenyl mit 2 bis 5 Kohlenstoffatomen, Alkinyl mit 2 bis 5 Kohlenstoffatomen oder für Cycloalkyl mit 3 bis 7 Kohlenstoffatomen steht,
wobei
R⁶ für Wasserstoff oder für geradkettiges oder verzweigtes Alkyl mit 1 bis 3 Kohlenstoffatoman steht,
R⁷ für einen jeweils geradkettigen oder verzweigten Alkyl mit 1 bis 8 Kohlenstoffatomen, Halogenalkyl mit 1 bis 4 Kohlenstoffatomen und 1 bis 9 gleichen oder verschiedenen Halogenatomen, Alkoxyalkyl, Alkoxyalkoxyalkyl oder Alkoxycarbonylalkyl mit jeweils 1 bis 4 Kohlenstoffatoman in den einzelnen Alkylteilen, Alkenyl mit 2 bis 4 Kohlenstoffatomen oder für gegebenenfalls einfach bis dreifach, gleich oder verschieden durch geradkettiges oder verzweigtes Alkyl mit 1 bis 3 Kohlenstoffatomen substituiertes Cycloalkyl mit 3 bis 7 Kohlenstoffatomen steht,
R⁸ für Wasserstoff oder für geradkettiges oder verzweigtes Alkyl mit 1 bis 3 Kohlenstoffatomen steht und
R⁹ für einen jeweils geradkettigen oder verzweigten Rest der Reihe Alkyl mit 1 bis 8 Kohlenstoffatomen, Halogenalkyl mit 1 bis 4 Kohlenstoffatomen und 1 bis 9 gleichen oder verschiedenen Halogenatomen, Alkoxyalkyl, Alkoxyalkoxyalkyl oder Alkoxycarbonylalkyl mit jeweils 1 bis 4 Kohlenstoffatomen in den einzelnen Alkylteilen, Alkenyl mit 2 bis 4 Kohlenstoffatomen oder für gegebenenfalls einfach bis dreifach, gleich oder verschieden durch geradkettiges oder verzweigtes Alkyl mit 1 bis 3 Kohlenstoffatomen substituiertes Cycloalkyl mit 3 bis 7 Kohlenstoffatomen steht.

4. Verfahren zur Herstellung von N-Aryl-Stickstoffheterocyclen dar allgemeinen Formel (I) gemäß Anspruch 1 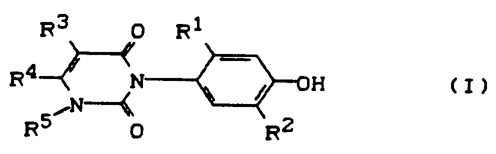 in welcher
R¹ für Wasserstoff oder Halogen steht,
R² für einen der Reste 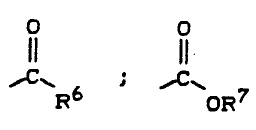 oder 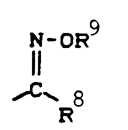 steht,
R³ für Wasserstoff steht und
R⁴ für Alkyl oder Halogenalkyl steht oder
R³ und R⁴ gemeinsam für einen zweifach verknüpften Alkandiylrest stehen und
R⁵ für einen jeweils geradkettigen oder verzweigten Rest der Reihe Alkyl, Alkenyl, Alkinyl, Halogenalkyl oder Cycloalkyl steht,
wobei
R⁶ für Wasserstoff oder Alkyl steht,
R⁷ für einen jeweils gegebenanfalls substituierten Rest der Reihe Alkyl, Alkenyl oder Cycloalkyl steht,
R⁸ für Wasserstoff oder Alkyl steht und
R⁹ für einen jeweils gegebenenfalls Substituierten Rest der Reihe Alkyl, Alkenyl oder Cycloalkyl steht
dadurch gekennzeichnet, daß man
a) Isocyanate der Formel (II), 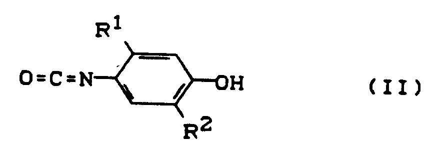 in welcher
R¹ und R² die oben angegebene Bedeutung haben,
mit β-Enaminoestern der Formel (III), 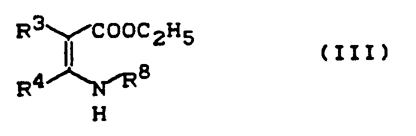 in welcher
R³ und R⁴ die oben angegebene Bedeutung haben und
R⁸ für Wasserstoff, oder einen jeweils geradkettigen oder verzweigten Rest der Reihe Alkyl, Alkanyl, Alkinyl, Halogenalkyl oder Cycloalkyl steht,
gegebenenfalls in Gegenwart eines Verdünnungsmittels und gegebenenfalls in Gegenwart eines Reaktionshilfsmittels umsetzt und gegebenenfalls anschließend
b) die so erhältlichen N-Aryl-Stickstoffheterocyclen der Formel (IV), 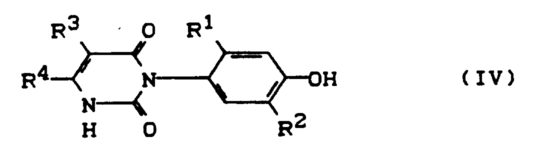 in welcher
R¹, R², R³ und R⁴ die oben angegebenen Bedeutungen haben,
mit Alkylierungsmitteln der Formel (V),
R⁵-E (V)
in welcher
R⁵ die oben angegebene Bedeutung hat und
E für eine elektronenanziehende Abgangsgruppe steht,
gegebenenfalls in Gegenwart eines Verdünnungsmittels und gegebenenfalls in Gegenwart eines Reaktionshilfsmittals umsetzt oder daß man
c) die mit Hilfe der erfindungsgemäßen Verfahren (a) oder (b) erhältlichen N-Aryl-Stickstoffheterocyclen dar Formel (Ia), 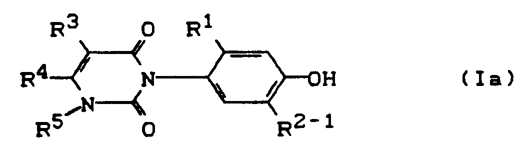 in welcher
R¹, R³, R⁴ und R⁵ die oben angegebenen Bedeutungen haben und
R²⁻¹ für einen Alkoxycarbonylrest steht,
zunächst mit Säure gegebenenfalls in Gegenwart eines Verdünnungsmittels an der Estergruppe im Arylteil verseift und in einer anschließenden 2. Stufe die so erhältlichen N-Aryl-Stickstoffheterocyclen der Formel (VI), 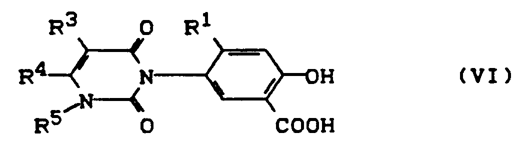 in welcher
R¹, R³, R⁴ und R⁵ die oben angegebenen Bedeutungen haben,
mit Alkoholen der Formel (VII),
R⁷-OH (VII)
in welcher
R⁷ die oben angegebene Bedeutung hat,
gegebenenfalls in Gegenwart eines Verdünnungsmittels und gegebenenfalls in Gegenwart eines Reaktionshilfsmittels verestert.

5. Herbizide Mittel, gekennzeichnet durch einen Gehalt an mindestens einem N-Aryl-Stickstoffheterocyclus der Formel (I) gemäß den Ansprüchen 1 bis 5.

6. Verfahren zur Bekämpfung von unerwünschten Pflanzen, dadurch gekennzeichnet, daß man N-Aryl-Stickstoffheterocyclen der Formel (I) gemäß den Ansprüchen 1 bis 5 auf unerwünschte Pflanzen und/oder ihren Lebensraum einwirken läßt.

7. Verwendung von N-Aryl-Stickstoffheterocyclen der Formel (I) gemäß den Ansprüchen 1 bis 5 zur Bekämpfung von unerwünschten Pflanzen,

8. Verfahren zur Herstellung von herbiziden Mitteln, dadurch gekennzeichnet, daß man N-Aryl-Stickstoffheterocyclen der Formel (I) gemäß den Ansprüchen 1 bis 5 mit Streckmitteln und/oder oberflächenaktiven Substanzen vermischt.

9. N-Aryl-Stickstoffheterocyclen der allgemeinen Formel (IV) 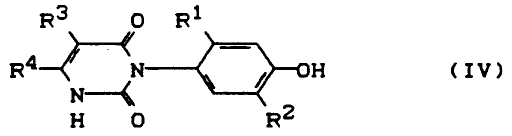 dadurch gekennzeichnet, daß
R¹ für Wasserstoff oder Halogen steht,
R² für einen der Reste 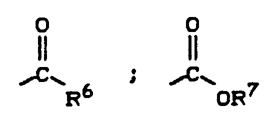 oder 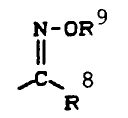 steht,
R³ für Wasserstoff steht und
R⁴ Alkyl oder Halogenalkyl steht oder
R³ und R⁴ gemeinsam für einen zweifach verknüpften Alkandiylrest stehen,
wobei
R⁶ für Wasserstoff oder Alkyl steht,
R⁷ für einen jeweils gegebenenfalls substituierten Rest der Reihe Alkyl, Alkenyl oder Cycloalkyl steht
R⁸ für Wasserstoff oder Alkyl steht und
R⁹ für einen jeweils gegebenenfalls substituierten Rest der Reihe Alkyl, Alkenyl oder Cycloalkyl steht.

10. N-Aryl-Stickstoffheterocyclen dar allgemeinen Formel (VI), 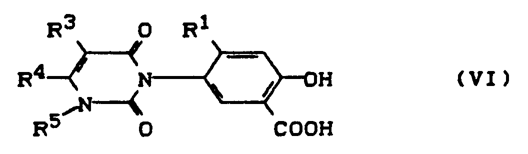 dadurch gekennzeichnet, daß
R¹ für Wasserstoff oder Halogen steht,
R³ für Wasserstoff steht und
R⁴ für Alkyl oder Halogenalkyl steht oder
R³ und R⁴ gemeinsam für einen zweifach ver-knüpften Alkandiylrest stehen und
R⁵ für einen jeweils geradkettigen oder verzweigten Rest der Reihe Alkyl, Halogenalkyl, Alkenyl, Alkinyl oder Cycloalkyl steht.
